(19) **Europäisches Patentamt**

**European Patent Office**

**Office européen des brevets**

(11) **EP 0 587 055 B1**

(12) **EUROPÄISCHE PATENTSCHRIFT**

(45) Veröffentlichungstag und Bekanntmachung des Hinweises auf die Patenterteilung:
**05.12.2001 Patentblatt 2001/49**

(51) Int Cl.⁷: **A61K 38/44**

(21) Anmeldenummer: **93114113.9**

(22) Anmeldetag: **03.09.1993**

(54) **Verwendung von Mangan-Superoxiddismutase (Mn-SOD) zur Herstellung von Arzneimitteln zur Behandlung von Erkrankungen in niedriger Dosierung**

Use of manganese superoxide dismutase (Mn-SOD) in low dosage for the production of pharmaca for the treatment of diseases

Utilisation de manganèse superoxyde dismutase (Mn-SOD) en bas dosage pour la production de médicaments pour le traitement de maladies

(84) Benannte Vertragsstaaten:
**AT BE CH DE DK ES FR GB GR IE IT LI LU MC NL PT SE**

(30) Priorität: **09.09.1992 DE 4229801**

(43) Veröffentlichungstag der Anmeldung:
**16.03.1994 Patentblatt 1994/11**

(73) Patentinhaber: **BIO-TECHNOLOGY GENERAL CORPORATION**
**Iselin, NJ 08830 (US)**

(72) Erfinder:
• **Blake, David R., Prof.**
**Droitwich, Worcs. WR9 7BE (GB)**

• **Dowling, Ewa J., Dr.**
**Chalfont St. Peter, Bucks. SL9 9EX (GB)**
• **Lillie, Christian, Dr.**
**A-1130 Wien (AT)**
• **Zöphel, Andreas, Dr.**
**A-3040 Neulengbach (AT)**

(74) Vertreter: **VOSSIUS & PARTNER**
**Postfach 86 07 67**
**81634 München (DE)**

(56) Entgegenhaltungen:
**EP-A- 0 070 656      EP-A- 0 284 105**
**CH-A- 676 990**

## Beschreibung

Als Folge verschiedener biochemischer Prozesse in biologischen Systemen (z.B. Redoxprozesse in der Atmungskette, Oxidationen im Cytoplasma) werden bekannterweise laufend $O_2$.-Radikale gebildet, die hochcytotoxisch sind und zu Gewebezerstörungen führen können. Bei pathologischen Situationen, z.B. im Verlauf rheumatisch bedingter Erkrankungen, werden Degradationen von Collagen und Synovialflüssigkeit durch solche Radikale diskutiert (Pasquier, C. et al., Inflammation 8, 27-32, 1984).

Eukaryotische Zellen enthalten hauptsächlich zwei Formen von Superoxiddismutasen (SOD), von denen die eine vornehmlich im Cytosol (Cu/Zn-SOD) und die andere vornehmlich in den Mitochondrien (Mn-SOD) vorliegen. In Lebermitochondrien wurde gefunden, daß das Mn-Enzym in der Matrix einschließlich der inneren Membran lokalisiert ist, obwohl die Mn-SOD auch im Cytosol der Leberzellen nachgewiesen wurde (McCord J.M. et al. In: Superoxide and Superoxide Dismutases (A.M. Michelson, J.M. McCord , I. Fridovich, eds.) Academic Press, N.Y., 129-138, 1977).

In Prokaryoten existiert neben einer Mn-SOD noch eine Fe-SOD. Letztere wurde auch in Algen und Protozoen sowie in einigen Pflanzenspezies nachgewiesen (Bridges, S.M., Salin, M.L., Plant Physiol. 68, 275-278, 1981).

Diese hochaktiven Enzyme katalysieren die Disproportionierung $O_2$.$^-$ +$O_2$.$^-$ +$2H^+ \rightarrow H_2O_2$+$O_2$ und verhindern durch diese Dismutation der Superoxidradikale deren Anreicherung und somit deren zellschädigende Wirkung. Neben dem endoplasmatischen Retikulum der Leber können die mitochondrialen Membranen als einer der wichtigsten Orte der $O_2$ Entstehung in tierischen Zellen angesehen werden, sodaß es nicht verwundert, daß Mitochondrien ihrer eigene, spezielle SOD(Mn-SOD) zur Verfügung haben.

Das Strukturgen einer prokaryotischen Mn-SOD (E.coli) wurde cloniert und das chromosomale sodA-Gen lokalisiert (Touati, D., J. Bact. 155, 1078-1087, 1983).

Die 699 bp lange Nukleotidsequenz einer mitochondrialen Hefe Mn-SOD konnte aufgeklärt und die Primärstruktur sowohl des Precursors als auch des maturen Proteins davon abgeleitet werden - mit Molekulargewichten von 26123 Da für den Precursor und 23059 Da für das mature Protein (Marres, C.A.M. et al., Eur. J. Biochem. 147, 153-161, 1985). Somit unterscheiden sich die Mn - und Cu/Zn-SOD (MG=14893, EP-A 138111) in ihren Molekulargewichten deutlich.

Die vollständige Aminosäuresequenz der Mn-SOD aus Menschenleber wurde von Barra, D. publiziert, wonach die hMn-SOD aus 196 Aminosäuren zusammengesetzt sein soll (Barra, D. et al. J. Biol. Chem. 259, 12595-12601, 1984). Die Human Cu/Zn-SOD aus Erythrocyten besteht demgegenüber aus 153 Aminosäuren (Jabusch, J.R., et. al. Biochemistry 19, 2310-2316, 1980) und zeigt keine Sequenzhomologien zur hMn-SOD (Barra, D. et al. siehe oben).

Generell wird den Superoxiddismutasen eine Schutzfunktion gegenüber bestimmten Entzündungsprozessen zugesprochen. Insbesondere soll einer Defizienz an Mn-SOD bei der Entwicklung der rheumatoiden Arthritis eine Bedeutung zukommen (Pasquier, C. et al., siehe oben). Ein protektiver Effekt der SOD gegen alkoholinduzierte Leberschädigung wird ebenfalls angenommen (Del Villano B.C. et al., Science 207, 991-993, 1980). Darüberhinaus soll die SOD ischemische Gewebe schützen (McCord, J.M. & Roy, R.S., Can. J. Physiol. Pharma 60, 1346-1352, 1982) bzw. kardioprotektiv wirken, in dem die SOD myocardialen Zellen bei Hypoxie bzw. bei durch Reperfusion bedingten Gewebeschädigungen Schutzfunktionen ausübt (Omar, B.A., McCord, J.M., J. Mol. Cell. Cardiol. 23, 149-159, 1991).

Es ist heute allgemein bekannt, daß freie Sauerstoffradikale bei den verschiedensten Zell- und Gewebeschädigungen eine zentrale Rolle spielen und insbesondere bei der Auslösung von Entzündungsprozessen einer der hauptsächlichen Faktoren darstellen.

Gemäß dieser Erkenntnis wurde versucht, die SOD als therapeutisches Mittel insbesondere bei Krankheiten einzusetzen, die mit Entzündungsprozessen einhergehen.

Es hat sich jedoch gezeigt, daß entweder die bisher im Milligramm-Bereich (mg/kg Körpergewicht) verwendeten Mn-SOD Mengen negative Effekte zeigte, wonach es zur Exazerbation des Entzündungsprozesses kommen kann, oder daß die Mn-SOD überhaupt keine Wirkung zeigte (Omar, B.A. McCord, J.M., Free Radical Biol. Med. 9, 473-478, 1990; Baret, A. et al., Biochem. Pharmacol. 33, 2755-2760, 1984). Entsprechend wird in der EP-A 284 105 vorgeschlagen, Mn-SOD in einer vorzugsweisen Dosierung zwischen 3 und 50 mg/kg Körpergewicht zur Behandlung entzündlicher Erkrankungen zu verwenden, oder wie es auch von Nimrod, A. et al., 1989, in: Medical, Biochemical and Chemical Aspects of Free Radicals, O. Hayaishi, E. Niki, M. Kondo, T. Yoshikawa, eds. Elsevier Science Publishers, B.V. Amsterdam pp. 743-746 beschrieben wird, wo ein Dosisbereich von 5 bis 50 mg/kg Körpergewicht angegeben wird. Aus der Publikation von Gorecki, M. et al., Free Rad. Res. Comms. Vols. 12-13, 401-410, 1991, ist bekannt, daß Mn-SOD bei einer Dosis von 100 mg/kg i.v. radioprotektiv und innerhalb eines Dosisbereichs von 8 bis 20 mg/kg i.v. bzw. bei 50 mg/kg s.c. antiinflammatorisch wirken soll.

Überraschenderweise wurde festgestellt, daß die Verwendung von Mn-SOD im niedrigen Dosisbereich von 1 bis 200 µg/kg Körpergewicht als Tagesdosis sehr effektiv war bei Erkrankungen, die durch freie Sauerstoffradikale verursacht werden, und daß nur in diesem Dosisbereich eine Zell- bzw. cytoprotektive Wirkung ohne nachteilige Effekte erhalten wurde. Bevorzugt wird ein Dosisbereich von 1 bis 100 µg/kg Körpergewicht, insbesondere von 1 bis 50 µg/kg Körpergewicht, wobei diese Dosisangaben bezogen sind auf eine Tagesdosis. Bei entzündlichen Erkrankungen, die direkt die Gelenke betreffen, beispielsweise arthritische Prozesse bzw. synovialen Endzündungsprozessen (Synovitis), haben sich Dosen von 1 bis 20 µg, insbesondere 1 bis 10 µg, ganz besonders 1 bis 5 µg an Mn-SOD, bezogen auf das Kniegelenkvolumen einer Ratte, als sehr wirksam erwiesen. Kniegelenke von adulten Ratten (ca. 200g) haben ein Volumen von durchschnittlich 100 µl (Miao, F.J.-P. et al., J. Pharm. Exp. Ther. 262, 889-895, 1992; Vadas, P. et al., Am. J. Pathol. 134, 807-811, 1989).

Die Wirksamkeit der erfindungsgemäßen niedrigen Dosierung von Mn-SOD war im Tierversuch hoch signifikant und es besteht kein Zweifel darüber, daß diese niedrige Dosierung auch zur Herstellung einer Arzneimittelpräpration zur therapeutischen oder prophylaktischen Behandlung von durch freie Sauerstoffradikale verursachten Erkrankungen, insbesondere Entzündungen, oder Zuständen beim Menschen anzuwenden ist.

Überraschenderweise wurde auch gefunden, daß die Mn-SOD in der erfindungsgemäßen Dosierung sehr viel effektiver ist als die Cu/Zn-SOD und daß hohe Dosen an Mn-SOD (> 200 µg/kg bzw. > 25 µg/100 µl Gelenkvolumen) und allgemein die Zn/Cu-SOD (100 µg/kg bzw. 5 bis 50 µg/kg) entweder zur Exazerbation der Krankheitsprozesse führten oder ineffektiv waren.

Bei der hier gefundenen Exazerbation der Zell- bzw. Gewebeschädigung bei hohen Mn-SOD Dosen oberhalb von 200 µg/kg bzw. oberhalb von 25 bis 50 µg/100 µl Gelenkvolumen bei Gelenkentzündungen wie z.B. arthritischen Erkrankungen (z.B. Synovitis) kann nur vermutet werden, daß diese nachteiligen Wirkungen in einer erhöhten Bildung von $H_2O_2$ durch die Disproportionierungsreaktion der Mn-SOD ihre Ursache haben könnten, wobei über Wechselwirkungen an reduzierten Übergangsmetallen (z.B. Fe, Mn) Hydroxylradikale gebildet werden könnten. Derartige Hydroxylradikale sind sehr reaktiv und könnten sich am Ort ihres Entstehens mit verschiedenen Molekülen rasch verbinden.

Denkbar ist auch, daß Sauerstoffradikale mit Stickoxyd (NO) zu Peroxynitrit ($NO_3^-$) reagieren , welches über die Reaktion $NO_3^- + H^+ \rightarrow NO_3H$ und gemäß der Folgereaktion $NO_3H \rightarrow OH. + NO_2.$ die Bildung von Hydroxylradikalen bedingt. Die durch ein übermäßiges Abfangen ("overscavenging") von $O_2.^-$ durch die Mn-SOD bedingten, verlängerten vasorelaxierenden Effekte des NO können zur Extravasation und Ödembildung führen, die für die inflammatorischen Effekte verantwortlich sein können, und die bei den hier aufgezeigten Untersuchungen mit hohen Dosen an Mn-SOD (> 200 µg/kg bzw. > 25-50 µg/100 µl Gelenkvolumen) festgestellt worden waren. Auch die Beteiligung der Mn-SOD an der reversiblen Reduktion von NO zum Nitroxylanion ist denkbar.

Hinsichtlich der durch die erfindungsgemäße Dosierung zu behandelnder Krankheiten und Zustände, die durch freie Sauerstoffradikale verursacht, mitverursacht oder bedingt werden oder bei denen freie Sauerstoffradikale unerwünschte Eigenschaften entfalten, zählen beispielsweise alle Arten von Entzündungsprozessen (wie z.B. synoviale Entzündungen, Morbus Crohn, Colitis rheumatische Prozesse), Gewebeschädigungen aufgrund vorgenomener medizinischer Eingriffe (z.B. Reperfusion und nachfolgende Ischämie) oder aufgrund von mechanischen, physikalischen (z.B. Strahlen) oder chemischen Einwirkungen, Organ- und Gewebetransplantationen oder arthritischen Prozessen Insbesondere umfassen die Entzündungen solche Prozesse, die perakut, akut, subakut, chronisch oder rezidivierend verlaufen als auch solche, die hinsichtlich ihrer Ausbreitung und Lokalisierung als lokalisierte, generalisierte oder metastasierende Entzündungen bekannt sind. Dazu zählen alle Arten der bekannten exsudativen, granulomatösen und proliferativen Entzündungen.

Die in der erfindungsgemäßen niedrigen Dosierung zu verwendende Mn-SOD kann vorzugsweise über die bekannten Methoden der DNA-Rekombination hergestellt werden, wobei das Verfahren gemäß der EP-A 282 899 bevorzugt wird.

Die erfindungsgemäß zu verwendende Mn-SOD kann in allen dem Fachmann geläufigen parenteralen oder enteralen bzw. systemisch oder topisch zu verwendenden Formulierungen vorliegen, wobei die systemische Applikation (z.B. i.v., i.m., s.c., i.a., i.p.) bevorzugt wird. Auch die dem Fachmann bekannten Depotformen und/oder mikroverkapselten Formen (z.B. in Liposomen) können für den erfindungsgemäßen Zweck sehr gut verwendet werden. Für die Herstellung der jeweiligen Darreichungsform werden die dem Fachmann bekannten Hilfs- und Trägerstoffe verwndet, wie beispielsweise NaCl, Na-Phosphat, Hydroxypropyl-β-cyclodestrin (HPBCD), Mannitol, Hydroxyethylcellulose.

Für die systemische Behandlung kann die Mn-SOD, vorzugsweise die gemäß der EP-A 282 899 herstellbare, rekombinante Mn-SOD, entweder als Bolus bzw. Einmaldosis in der erfindungsgemäßen Dosierung an oder in das erkrankte Gewebe (i.m., s.c., i.p.) oder auf andere Weise in den Blutstrom (i.v., i.a.) oder auf dem Weg einer Infusion appliziert werden, wobei tägliche Einmal- und Mehrfachgaben oder Gaben an aufeinanderfolgenden Tagen, wöchentliche oder monatliche Applikationen, je nach Art, Schweregrad und Ansprechrate der Erkrankung, in Betracht kommen.

LEGENDEN ZU DEN FIGUREN

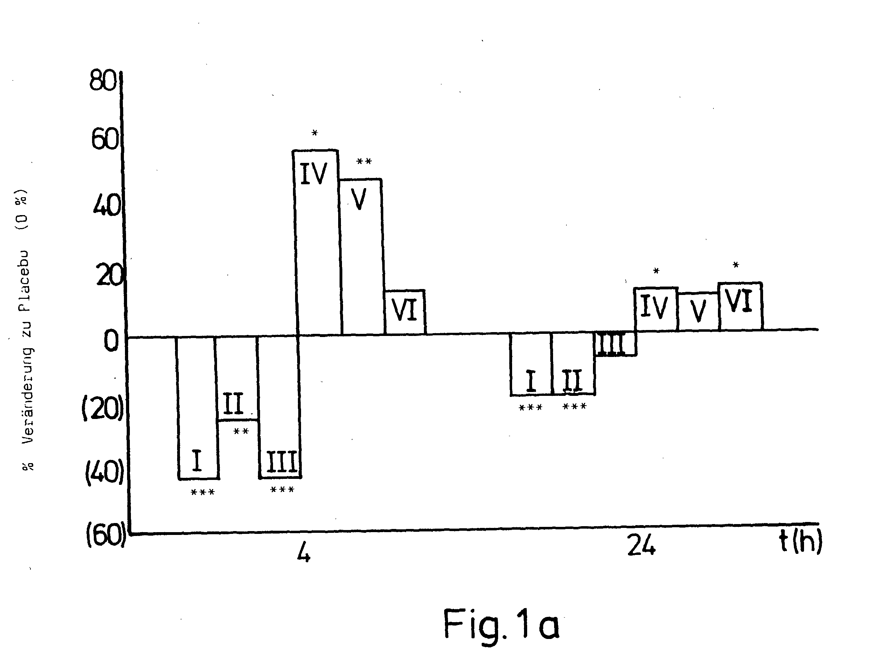

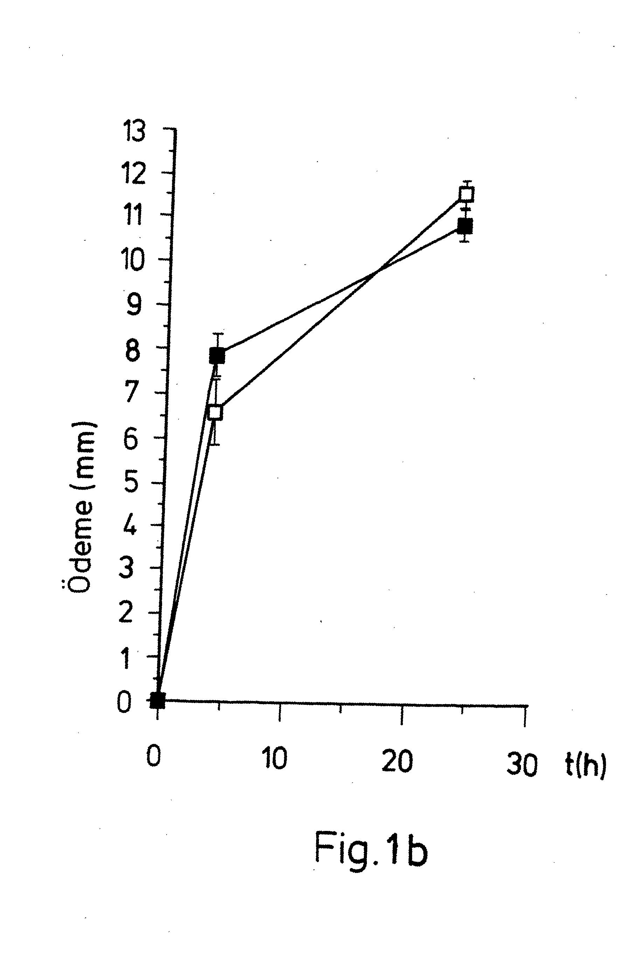

Fig. 1:        Dosis-Reaktion von (a) rh Mn-SOD und (b) Cu/Zn-SOD bei Ödemen im FCA-mediiertem Pfoten-Modell

a: Die römischen Zahlen repräsentieren diejenigen Tiere, die mit verschiedenen Dosen an rh Mn-SOD intraperitoneal (i.p.) behandelt wurden (KG= Körpergewicht) I= 50 µg/kg KG, II= 100 µg/kg KG, III= 200 µg/kg KG, IV= 400 µg/kg KG, V= 800 µg/kg KG, VI= 2 mg/kg KG. Jeder Wert zeigt die gemittelten Daten von jeweils 6 Tieren (nach 4 und nach 24 Stunden) und ist ausgedrückt als prozentuale Änderung gegenüber Placebo [Phosphatpuffer] (0 %) *= p< 0,05, **= p< 0,01 , ***= p< 0,001 als signifikante Unterschiede zur Placebo-Gruppe (ungepaarter Student t-Test)

b: Die Symbole repräsentieren die mit Lösungsmittel [Phosphatpuffer] (□) oder mit Cu/Zn-SOD in der Dosis von 100 µg/kg KG (■) intraperitoneal (i.p.) behandelten Tiere. Jeder Punkt zeigt die gemittelten Daten von jeweils 6 Tieren, die vertikalen Balken zeigen die Standartabweichung.

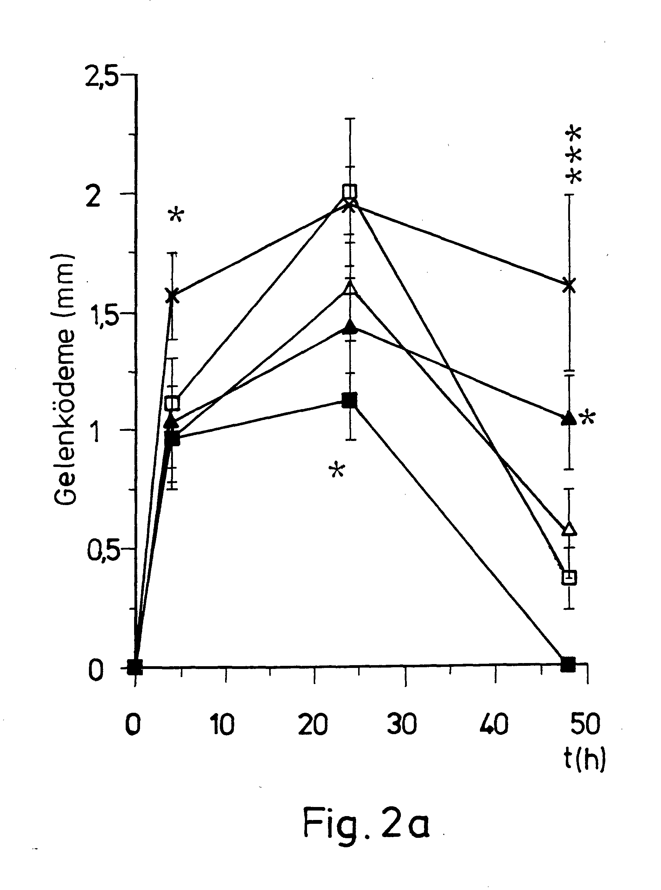

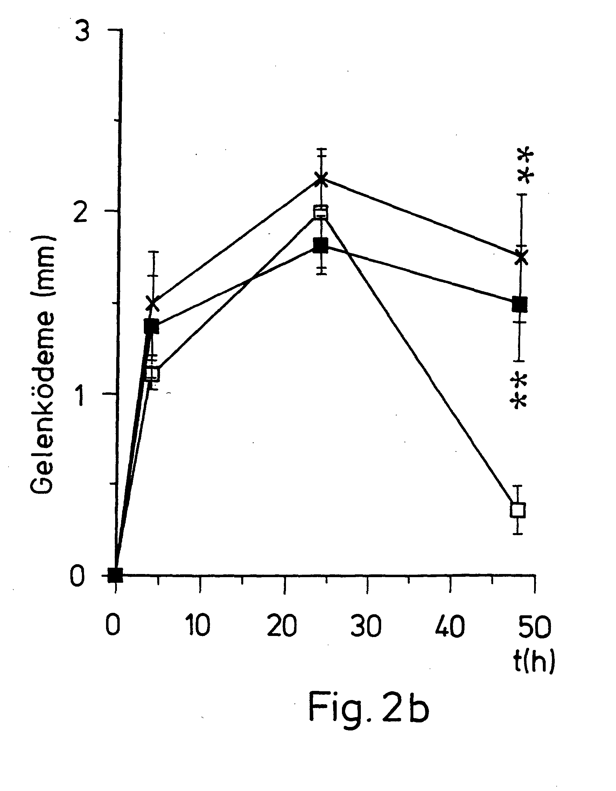

Fig. 2: Dosis-Raktion von (a) rh Mn-SOD und (b) Cu/Zn-SOD bei Gelenködemen beim Carrageenan-induziertem Synovitis Modell

a: Die Symbole repräsentieren die mit Lösungsmittel [physiologische NaCl-Lösung] (□) oder rh Mn-SOD in der Dosis von 5 µg (□), 12,5 µg (△), 25 µg (△) oder 50 µg (X) intraartikulär (i.a.) behandelten Tiere. Jeder Punkt zeigt die gemittelten Daten von 6 oder 7 Tieren; die vertikalen Balken zeigen die Standartabweichung.

b: Die Symbole repräsentieren die mit Lösungsmittel [physiologische NaCl-Lösung] (□) oder Cu/Zn-SOD in der Dosis von 5 µg (□) oder 50 µg (X) intraartikulär (i.a.) behandelten Tiere. Jeder Punkt zeigt die gemittelten Daten von 5 oder 6 Tieren; die vertikalen Balken zeigen die Standartabweichung. *= p< 0,05 , **= p< 0,01, ***= p< 0,001 als signifikante Unterschiede zu den mit Lösungsmittel injizierten Tieren (ungepaarter Student t-Test).

Die folgenden Beispiele sollen die Erfindung näher erläutern. Alle Ergebnisse sind als mittlerer + Standartfehler der Mittelwerte (Standartabweichung) dargestellt. Die Signifikanzanalyse wurde nach dem ungepaarten Student t-Test berechnet. Eine Wahrscheinlichkeit von 0,5 wurde als minimales Signifikanzniveau festgelegt.

Beispiel 1: Adjuvant-induzierte Pfotenödeme bei der Ratte

Bei Gruppen von jeweils sechs männlichen Wistar Ratten (150 bis 200 g) wurden durch eine einzige subplantare Injektion (50 µl) von Freund's Complete Adjuvant (FCA, hitzegetötetes Mycobacterium tuberculosis, Humanstämme C, DT und PN gemischt [Central Veterinary Laboratories, MAFF, Weybridge, Surrey, U.K., gefriergetrocknet] suspendiert in Flüssigparaffin BP/10mg/ml) Fußödeme induziert. Die Ratten wurden 6 Tage vorher mit 75 µl FCA, ins Genick appliziert, immunisiert. Humane rekombinante Mangan Superoxiddismutase (rh Mn-SOD) mit der spezifischen Aktivität von 3625 U/mg Protein (hergestellt gemäß EP-A 282 899 bzw. bezogen von Bender & Co. GesmbH, Wien) wurde in Phosphatpuffer (0,06M, ph 7,8) in Endkonzentrationen von 50,100,200,400,800 µg/kg und 2 mg/kg gelöst und eine Stunde vor der FCA-Induktion intraperitoneal in eine Pfote der einen Gruppe von Ratten (n=6) appliziert.

Eine zusätzliche Gruppe an Ratten (n=6) erhielt eine Stunde vor der FCA-Induktion Cu/Zn-SOD (aus Human Erythrozyten, spezifische Aktivität 3610 U/mg Protein, Sigma Chemical Company, U.K.), gelöst in Phosphatpuffer wie oben angegeben (100 µg/kg Körpergewicht). Die Pfotenödeme wurden durch Messen des Pfotenumfangs (in mm) nach 4 und 24 Stunden der FCA-Induktion bestimmt und als Größendifferenz zwischen den inokulierten und den nicht-inokulierten Pfoten ausgedrückt.

Anhand dieses exemplarischen Modells einer zellvermittelten Hypersensitivitätsreaktion wurde der Dosis-Reaktion-Effekt von rh Mn-SOD bei Ödemen untersucht, die sich 4 und 24 Stunden nach der FCA-Induktion entwickelt hatten. Nach der FCA-Induktion entwickelten sich die Pfotenödeme innerhalb der anfänglichen 4 Stunden schnell und erreichten ihre maximale Ausdehnung nach 24 Stunden. In Figur la wird die vollständige Serie der durchgeführten Experimente dargestellt, in denen die Effekte von rh Mn-SOD als prozentuale Änderung gegenüber Placebo berechnet wurden. Eine Vorbehandlung mit rh Mn-SOD resultierte in einem "parabolischen" Dosis-Reaktions-Effekt, wobei höhere rh Mn-SOD Dosen (400-800 µg/kg und 2 mg/kg) eine pro-inflammatorische Reaktion nach sowohl 4 als auch, mit geringerem Ausmaß, nach 24 Stunden. Eine signifikante Reduktion der Ödeme wurde im niederen Dosisbereich der rh Mn-SOD (50,100,200 µg/kg) festgestellt, mit mittleren Reduktionswerten bei 4 Stunden von 43% (p< 0,001) bzw. 25% (p< 0,01.) und 43% (p< 0,001) und 19% (p< 0,001 ) bei den beiden niederen rh Mn-SOD Dosen (50,100 µg/kg) nach 24 Stunden. Bei den mit der Cu/Zn-SOD behandelten Gruppen waren keine signifikanteh Veränderungen bei der Ödementwicklung festzustellen (Figur 1b).

Beispiel 2: Carrageenan-Induziertes Synovitis Modell bei der Ratte

Bei Gruppen von jeweils sechs bis sieben männlichen Wistar Ratten (150 bis 200g) wurden durch eine einzige intraartikulär Injektion (50 μl) von Carrageenan (1% w/v in steriler physiologischer Kochsalzlösung; Carrageenan Viscarin 402 von Marine Colloids Inc., Springfield, USA) in das Kniegelenk eine Entzündung induziert. Zu spezifischen Zeitpunkten nach der Induktion (0, 4, 24, 48 Stunden) wurden die Gelenködeme durch Messung der Kniebreite (mm) bestimmt und als Differenz zwischen injiziertem und contralateralen, nicht-injiziertem Kniegelenk ausgedrückt. In jeweils ein Kniegelenk wurden Gruppen von Ratten (n=6-7) eine einzige intraartikuläre Injektion (50 μl) von entweder a) Carrageenan, b) Carrageenan + rh Mn-SOD (5, 12,5 , 25, 50 μg; Aktivität, Lösung und Herkunft der rh Mn-SOD wie unter Beispiel 1 angegeben), c) rh Mn-SOD (50 μg), d) Carrageenan + Cu/Zn-SOD (5,50 μg; Aktivität, Lösung und Herkunft der Cu/Zn-SOD wie unter Beispiel 1 angegeben) oder e) Saline (physiologische Kochsalzlösung) appliziert.

Die intraartikuläre (i.a.) Injektion (50 μl) von 1% w/v Carrageenan in ein Kniegelenk der Ratten ließ ein gleichmäßiges Ansteigen der Gelenködeme entstehen, welche ihre maximale Ausdehnung nach 24 Stunden erreichten. Die Behandlung mit rh Mn-SOD mit Dosen von 25 und 50 μg (i.a.) resultierte in einer ausgeprägten Exazerbation der Ödemreaktion bis 48 Stunden nach Induktion (Figur 2a). Eine signifikante Reduktion der Ödembildung (44%, $p < 0,05$) wurde nur mit der niedrigen Dosis an rh Mn-SOD (5 μg) beim Zeitpunkt 24 Stunden festgestellt. Ungünstige Wirkungen auf den Gelenkumfang nach Gabe von entweder rh Mn-SOD (50 μg) oder nur Saline traten nicht auf.

Jedoch wurde in der mit Cu/Zn-SOD behandelten Gruppe bis 48 Stunden nach der Injektion ein deutlicher pro-inflammatorischer Effekt festgestellt.

Beispiel 3: Mn-SOD Formulierungen

Folgende Lösungen sind für die erfindungsgemäße Verwendung der Mn-SOD, z.B. für die i.v., i.m., s.c., i.a., i.p., Enema und topische Anwendungen, geeignet:

| 1. | MnSOD | 0,1 - 20 mg/ml |
|----|-------|----------------|
|    | NaCl | 150 mM |
|    | Na-Phosph. | 10 mM |
|    | pH | 5,8 - 7,5 |

| 2. | MnSOD | 0,1 - 20 mg/ml |
|----|-------|----------------|
|    | HPBCD | 6 % |
|    | Na-Phosph. | 10 mM |
|    | pH | 5,8 - 7,5 |

Insbesondere für Enema oder topische Anwendung ist die folgende Formulierung geeignet:

| 3. | MnSOD | (0,15 - 3 mg/ml) in Hydrogel (1,75 % Hydroxyethylcellulose, 0,2 % Methylparaben als Konservans). |
|----|-------|---------------------------------------------------------------------------------------------------|

**Patentansprüche**

1. Verwendung von Mangan-Superoxiddismutase (Mn-SOD) zur Herstellung einer Arzneimittelpräparation zur therapeutischen oder prophylaktischen Behandlung von durch freie Sauerstoffradikale verursachten, mitverursachten oder bedingten Erkrankungen oder Zuständen, enthaltend 1 bis 200 μg an Mn-SOD, bezogen auf eine Tagesdosis pro kg Körpergewicht eines Säugetiers einschließlich eines Menschen.

2. Verwendung nach Anspruch 1, **dadurch gekennzeichnet, daß** die Präparation 1 bis 100 μg/kg Körpergewicht an Mn-SOD, enthält.

3. Verwendung nach Anspruch 1, **dadurch gekennzeichnet, daß** die Präparation 1 bis 50 μg/kg Körpergewicht an Mn-SOD enthält.

4. Verwendung nach einem der Ansprüche 1 bis 3, zur Herstellung einer Arzneimittelpräparation zur Behandlung von Gewebeschädigungen.

5. Verwendung nach Anspruch 4 zur Herstellung einer Arzneimittelpräparation zur Behandlung von Entzündungsprozessen.

6. Verwendung nach Anspruch 4 zur Herstellung einer Arzneimittelpräparation zur Behandlung von Geweben und Organen nach Transplantationen.

7. Verwendung von Mangan-Superoxiddismutase (Mn-SOD) zur Herstellung einer Arzneimittelpräparation zur Behandlung von entzündlichen Erkrankungen an Gelenken, enthaltend 1 bis 20 µg an Mn-SOD, bezogen auf ein Kniegelenkvolumen von 100 µl.

8. Verwendung nach Anspruch 7, **dadurch gekennzeichnet, daß** die Präparation 1 bis 10 µg an Mn-SOD enthält.

9. Verwendung nach Anspruch 8, **dadurch gekennzeichnet, daß** die Präparation 1 bis 5 µg an Mn-SOD enthält.

10. Verwendung nach einem der Ansprüche 1 bis 9 zur Herstellung einer Arzneimittelpräpration für die systemische Applikationsweise oder auf Basis von Depotformen.

11. Pharmazeutische Zusammensetzung in Form einer Dosiseinheit enthaltend Mn-SOD als aktives Mittel und ein oder mehrere pharmazeutische Hilfs- und Trägerstoffe, worin jede Dosiseinheit weniger als 14 mg an Mn-SOD enthält.

12. Pharmazeutische Zusammensetzung nach Anspruch 11, **dadurch gekennzeichnet, daß** in der Dosiseinheit weniger als 7 mg an Mn-SOD enthalten sind.

13. Pharmazeutische Zusammensetzung nach Anspruch 11, **dadurch gekennzeichnet, daß** in der Dosiseinheit weniger als 3,5 mg an Mn-SOD enthalten sind.

14. Pharmazeutische Zusammensetzung nach Anspruch 11, **dadurch gekennzeichnet, daß** in der Dosiseinheit weniger als 1,2 mg an Mn-SOD enthalten sind.

15. Pharmazeutische Zusammensetzung nach Anspruch 11, **dadurch gekennzeichnet, daß** in der Dosiseinheit weniger als 0,6 mg an Mn-SOD enthalten sind.

16. Pharmazeutische Zusammensetzung nach Anspruch 11, **dadurch gekennzeichnet, daß** in der Dosiseinheit weniger als 0,3 mg an Mn-SOD enthalten sind.


**Claims**

1. Use of manganese superoxide dismutase (Mn-SOD) for the production of a pharmaceutical preparation for the therapeutic or prophylactic treatment of diseases or conditions caused, partly caused or triggered by oxygen free radicals, which contains 1 to 200 µg Mn-SOD, based on a daily dosage per kg of body weight of a mammal including humans.

2. Use according to claim 1 **characterised in that** the preparation contains 1 to 100 µg/kg of body weight of Mn-SOD.

3. Use according to claim 1 **characterised in that** the preparation contains 1 to 50 µg/kg of body weight of Mn-SOD.

4. Use according to any one of claims 1 to 3 for the production of a pharmaceutical preparation for the treatment of tissue damage.

5. Use according to claim 4 for the production of a pharmaceutical preparation for the treatment of inflammatory processes.

6. Use according to claim 4 for the production of a pharmaceutical preparation for the treatment of tissue and organs after transplantation.

**7.** Use of manganese superoxide dismutase (Mn-SOD) for the production of a pharmaceutical preparation for the treatment of inflammatory diseases of joints, containing 1 to 20 µg Mn-SOD per volume of a knee joint of 100 µl.

**8.** Use according to claim 7 **characterised in that** the preparation contains 1 to 10 µg Mn-SOD.

**9.** Use according to claim 8 **characterised in that** the preparation contains 1 to 5 µg Mn-SOD.

**10.** Use according to any one of claims 1 to 9 for the production of a pharmaceutical preparation for systemic application or on the basis of deposit forms.

**11.** Pharmaceutical composition in the form of a dosage unit containing Mn-SOD as active agent and one or more pharmaceutical adjuvants and carrier substances, wherein each dose unit contains less than 14 mg Mn-SOD.

**12.** Pharmaceutical composition according to claim 11 **characterised in that** the dosage unit contains less than 7 mg Mn-SOD.

**13.** Pharmaceutical composition according to claim 11 **characterised in that** the dosage unit contains less than 3.5 mg Mn-SOD.

**14.** Pharmaceutical composition according to claim 11 **characterised in that** the dosage unit contains less than 1.2 mg Mn-SOD.

**15.** Pharmaceutical composition according to claim 11 **characterised in that** the dosage unit contains less than 0.6 mg Mn-SOD.

**16.** Pharmaceutical composition according to claim 11 **characterised in that** the dosage unit contains less than 0.3 mg Mn-SOD.

**Revendications**

**1.** Utilisation de manganèse superoxyde dismutase (Mn-SOD) pour la production d'une préparation pharmaceutique pour le traitement thérapeutique ou prophylactique de maladies ou d'états provoqués, partiellement provoqués ou déclenchés par des radicaux libres d'oxygène, qui contient 1 à 200 µg de Mn-SOD, sur la base d'un dosage journalier par kg de poids de corps d'un mammifère et notamment l'être humain.

**2.** Utilisation suivant la revendication 1, **caractérisée en ce que** la préparation contient 1 à 100 µg/kg de poids de corps de Mn-SOD.

**3.** Utilisation suivant la revendication 1, **caractérisée en ce que** la préparation contient 1 à 50 µg/kg de poids de corps de Mn-SOD.

**4.** Utilisation suivant l'une quelconque des revendications 1 à 3, pour la production d'une préparation pharmaceutique pour le traitement d'une détérioration tissulaire.

**5.** Utilisation suivant la revendication 4 pour la production d'une préparation pharmaceutique pour le traitement de processus inflammatoires.

**6.** Utilisation suivant la revendication 4 pour la production d'une préparation pharmaceutique pour le traitement de tissu et d'organes après une transplantation.

**7.** Utilisation de manganèse superoxyde dismutase (Mn-SOD) pour la production d'une préparation pharmaceutique pour le traitement d'affections inflammatoires d'articulations, contenant 1 à 20 µg de Mn-SOD par volume d'une articulation de genou de 100 µl.

**8.** Utilisation suivant la revendication 7, **caractérisée en ce que** la préparation contient 1 à 10 µg de Mn-SOD.

**9.** Utilisation suivant la revendication 8, **caractérisée en ce que** la préparation contient 1 à 5 µg de Mn-SOD.

**10.** Utilisation suivant l'une quelconque des revendications 1 à 9 pour la production d'une préparation pharmaceutique pour une application systémique ou sur la base de formes de dépôt.

**11.** Composition pharmaceutique sous la forme d'une unité de dosage contenant de la Mn-SOD comme agent actif et un ou plusieurs adjuvants et substances de support pharmaceutiques, dans laquelle chaque dose unitaire contient moins de 14 mg de Mn-SOD.

**12.** Composition pharmaceutique suivant la revendication 11, **caractérisée en ce que** l'unité de dosage contient moins de 7 mg de Mn-SOD.

**13.** Composition pharmaceutique suivant la revendication 11, **caractérisée en ce que** l'unité de dosage contient moins de 3,5 mg de Mn-SOD.

**14.** Composition pharmaceutique suivant la revendication 11, **caractérisée en ce que** l'unité de dosage contient moins de 1,2 mg de Mn-SOD.

**15.** Composition pharmaceutique suivant la revendication 11, **caractérisée en ce que** l'unité de dosage contient moins de 0,6 mg de Mn-SOD.

**16.** Composition pharmaceutique suivant la revendication 11, **caractérisée en ce que** l'unité de dosage contient moins de 0,3 mg de Mn-SOD.

Fig.1a

Fig.1b

Fig. 2a

Fig. 2b